# EUROPÄISCHE PATENTSCHRIFT

(11) **EP 3 417 732 B1**
(45) Veröffentlichungstag und Bekanntmachung des Hinweises auf die Patenterteilung: **20.01.2021**
(21) Anmeldenummer: 18178590.8
(22) Anmeldetag: 19.06.2018
(51) Int. Cl.: A43B 7/14, A41B 11/02, A61F 13/06, A61F 13/08

(54) **SCHUH MIT EINER FUSSSTIMULIERENDEN VORRICHTUNG**
SHOE WITH A FOOT STIMULATING DEVICE
CHAUSSURE DOTÉE D'UN DISPOSITIF STIMULANT LE PIED

(30) Priorität: 22.06.2017 DE 102017113846
(43) Veröffentlichungstag der Anmeldung: 26.12.2018
(73) Patentinhaber: Hero GmbH & Co. KG, 84048 Mainburg (DE)
(72) Erfinder: Haimerl, Ewald, 84048 Mainburg (DE)
(74) Vertreter: Hartig, Michael

(56) Entgegenhaltungen:
- DE-A1- 3 812 186
- DE-C- 441 042
- DE-T2- 69 508 513
- GB-A- 527 235
- US-A1- 2013 192 091

## Beschreibung

Die Erfindung betrifft einen Schuh mit einer den Fuß stimulierenden Vorrichtung, eine Sohle und einen Strumpf. Idealerweise bilden der Fuß und der Schuh eines Trägers eine funktionelle Einheit, die durch die grundsätzliche Passform des Schuhs und durch eine zusätzliche Verschlussmöglichkeit, wie beispielsweise eine Schnürung oder eine oder mehrere Schnallen, gebildet wird. Der Schuh soll einerseits den Fuß vor äußeren Einflüssen, wie beispielsweise Kälte, Nässe, Verletzungen schützen und andererseits seine physiologischen Funktionen, wie die Gehbewegung einschließlich des natürlichen Abrollens möglichst wenig behindern.

Auch bei einem weitgehend optimierten Schuh kann nicht verhindert werden, dass der Schuh aufgrund seiner Konstruktion, der gewählten Materialien und seiner Funktion die natürliche Funktion und natürliche Umgebung des Fußes beeinflusst und ggfs. zumindest geringfügig einschränkt.

GB527235 A offenbart eine Fußgewölbestütze zur Verwendung mit Schuhen zur Vermeidung und Linderung von Fußbeschwerden wie Spreiz- und Senkfuß. Weiter wird ein Leder- oder ähnliches Band offenbart, das entsprechend der Form des Fußes geschnitten wird, wobei zwei mit Ösen-Reihen versehene Seitenklappen durch das Futter des Schuhs gezogen werden können, nachdem geeignete Öffnungen in diesem vorgesehen wurden.

DE3812186 A1 offenbart eine Sandale mit einer Sohle, an der mindestens ein Band verankert ist, wobei mindestens eines der Bänder an der Sohle unter der oberen Säule eines Fußes verankert ist, die von dem Kahnbein, den ersten, zweiten und dritten Keilbeinen und den ersten, zweiten und dritten Mittelfußknochen gebildet ist.

US 2013/192091 A1 offenbart einen Schuhartikel, der eine Sohlenstruktur und ein Oberteil umfasst. Die Sohlenstruktur hat einen Randbereich und einen zentralen Bereich, der sich vom Randbereich nach innen erstreckt. Das Oberteil dient zur Aufnahme eines Fußes eines Trägers und hat eine äußere Schicht und eine innere Schicht. Die äußere Schicht erstreckt sich von einem Halsbereich des Oberteils zu einem unteren Bereich des Oberteils, und die Außenschicht ist an dem Umfangsbereich der Sohlenstruktur befestigt. Die innere Schicht befindet sich innerhalb der äußeren Schicht und erstreckt sich vom Halsbereich bis zum unteren Bereich. Die innere Schicht liegt neben der äußeren Schicht und die innere Schicht ist (a) im zentralen Bereich der Sohlenstruktur befestigt und (b) zwischen dem Halsbereich und dem zentralen Bereich der Sohlenstruktur nicht befestigt.

DE441042 C offenbart ein hygienisch-orthopädisches Schuhwerk mit beiderseitig angeordneten, den Fuß stützenden Einsatzteilen, die auf die Oberseite des Fußgewölbes geführt und untereinander und mit dem Oberleder verschlossen werden, wobei die Einsatzteile auf der Oberseite des Fußes sich gegenseitig durchdringen und jeder Teil mit der entgegengesetzten Seite des Oberleders verbunden wird, wobei einer der Einsatzteile oder beide in an sich bekannter Weise hängemattenartig unter dem Fuß durchgezogen sein können.

DE69508513 T2 offenbart ein Unterschenkelschutzbekleidungsstück, das auf der Oberfläche eines menschlichen Körpers komprimierend zu tragen ist und eine schlauchartige Form von der Nähe der Region knapp unter dem Kniegelenk bis zur Nähe des Knöchels aufweist. Das Kleidungsstück weist einen Abschnitt mit einer starken Dehnungskraft auf, der mindestens einen Teil des Fersenhöckers über die Sohle bedeckt und sich bis in die Nähe des Bereichs knapp unter dem Kniegelenk erstreckt, der mindestens einen Teil der beiden Seiten des Unterschenkelbereichs bedeckt, und einen Abschnitt mit einer schwachen Dehnungskraft in dem Abschnitt, der mindestens ein Drittel des Bereichs des Abschnitts mit der schlauchartigen Form unterhalb des Kniegelenks aufweist, wobei der Unterschenkelbereich verstärkt wird, um Schutz vor der Knöchelverstauchung oder deren Wiederholung zu bieten.

Es ist die Aufgabe der Erfindung, diese Einschränkungen des Fußes, die zu einer unphysiologischen Belastung und zur Ermüdung führen können, durch Stimulation bestimmter Bereiche des Fußes auszugleichen oder zu reduzieren.

Die Aufgabe wird durch die Gegenstände mit den Merkmalen der unabhängigen Ansprüchen gelöst. Vorteilhafte Ausführungsformen sind Gegenstand der abhängigen Ansprüche.

Gemäß einem Aspekt wird die Aufgabe durch einen Schuh gelöst, der eine bandförmige Vorrichtung aufweist, die an einer Innenseite des Schuhs von einem Ort am oberen Schaftende zum Schuhboden verläuft und auf der gegenüberliegenden Innenseite des Schuhs vom Schuhboden in Richtung des oberen Schaftendes verläuft, wobei die bandförmige Vorrichtung eingerichtet ist, auf einen im Schuh angeordneten Fuß einen Kontakt durch Druck auszuüben. Die bandförmige Vorrichtung kann einen, zwei oder mehrere bandförmige Abschnitte aufweisen. Eine einteilige bandförmige Vorrichtung kann an einer Innenseite des Schuhs von einem Ort am oberen Schaftende zum Schuhboden verlaufen, den Schuhboden queren und auf der gegenüberliegenden Innenseite des Schuhs in Richtung des oberen Schaftendes verlaufen. Ein oder beide Enden bzw. Abschnitte der bandförmigen Vorrichtung erstrecken sich bis zum oberen Endes des Schaftes. Zumindest ein Ende der bandförmigen Vorrichtung oder eines Abschnitts derselben kann jedoch auch am Schuhboden enden und dort am Schuh oder an der Innensohle festgelegt sein, Die bandförmige Vorrichtung kann den Schuhboden in einem Winkel zwischen 20° und 90° (d.h. beispielsweise mit 20°, 30°, 40°, 50° 60°, 70°, 80° oder 90°) zur Längsrichtung des Schuhs queren. Wenn sich beide Enden der bandförmigen Vorrichtung oder der Abschnitte vom Schuhboden auf den gegenüberliegenden Innenseiten des Schuhs in Richtung des Schaftrandes erstrecken, so verläuft gemäß einer Ausführungsform der auf der Großzehenseite des Schuhs verlaufende Abschnitt der bandförmigen Vorrichtung vor der Position des Innenknöchels eines im Schuh angeordneten Fußes, während der auf der Kleinzehenseite des Schuhs verlaufende Abschnitt der bandförmigen Vorrichtung hinter der Position des Außenknöchels des im Schuh angeordneten Fußes nach oben verläuft.

Gemäß einer Ausführungsform verlaufen beide Abschnitte der bandförmigen Vorrichtung zumindest im oberen Teil des Schuhs im Wesentlichen senkrecht nach oben in Richtung des Schaftrandes. Es ist jedoch auch denkbar, dass einer oder beide Abschnitte unter einem Winkel zwischen 0° und 45° im Verhältnis zur Senkrechten auf die Sohle des Schuhs nach oben verlaufen bzw. sich in dieser Richtung erstrecken, Der Winkel kann insbesondere zwischen 20° und 70°, zwischen 30° und 60° oder zwischen 35° und 55° (Grad) liegen und für die Abschnitte verschieden sein. Die Abschnitte erstrecken sich vorzugsweise geradlinig in die angegebenen Richtungen, könnten aber auch einen gekrümmten oder gebogenen Verlauf aufweisen.

Durch die im Schuh vorgesehene bandförmige Vorrichtung gemäß der vorliegenden Erfindung können im Bereich der bandförmigen Vorrichtung liegende Bereiche des Fußes durch Ausübung eines Drucks stimuliert und damit die Funktionen des Fußes und des Unterschenkels, die in einem herkömmlichen Schuh beeinträchtigt werden oder zum Erliegen kommen, über die gesamte Tragezeit eines Schuhs aktiviert werden. Der ausgeübte Druck kann abhängig von der Ausführung der bandförmigen Vorrichtung, insbesondere der Dicke bzw. Stärke derselben, für den Träger des Schuhs kaum wahrnehmbar oder deutlich spürbar sein. Auch kann der durch die bandförmige Vorrichtung ausgeübte Druck in einem oder mehreren Bereichen der bandförmigen Vorrichtung hinsichtlich der Stärke variieren. Verläuft die bandförmige Vorrichtung beispielsweise im Schuh über den Bereich der Achillessehne, kann die bandförmige Vorrichtung an dieser Stelle dünner gestaltet oder ausgespart sein, um Reibungen am Fuß zu vermeiden, Diese Bereiche können beispielsweise eine Länge von 1cm bis 5cm, insbesondere 1cm, 2cm, 3cm, 4cm, 5cm oder mehr aufweisen,

Die bandförmige Vorrichtung ist so im Schuh angeordnet, dass sie auf Muskelfaszienzüge des Fußes einen Druck ausübt. Die bandförmige Vorrichtung ist so im Schuh angeordnet, dass sie entlang einer oder mehrerer Muskelfaszienzüge des Fußes verläuft.

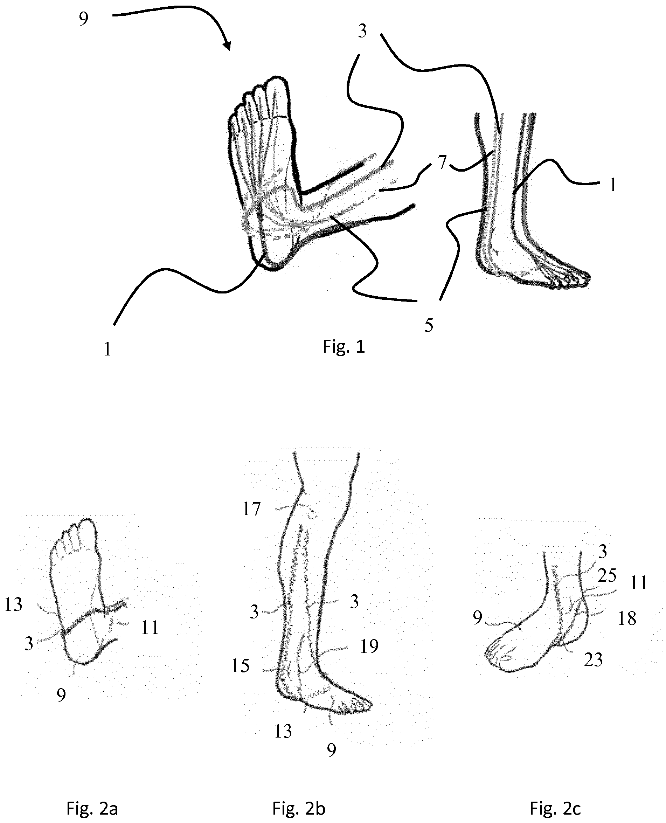

Der menschliche Körper weist eine Vielzahl von Muskel-Faszienzügen auf, mit welchen der Halte- und Bewegungsapparat des menschlichen Körpers kontrolliert bewegt und verspannt wird. Die Muskel-Faszienzüge ermöglichen es dem Menschen, aufrecht zu stehen, sich unterschiedlichen Situationen anzupassen, in verschiedenen Stellungen Arbeit zu verrichten und Leistungen zu erbringen. Die wichtigsten Muskel-Faszienzüge, die den Körper überziehen, liegen im Fuß eng beieinander und können dort ihren Anfang nehmen. Bedeutende Muskel-Faszienzüge sind die oberflächliche Rückenlinie 1, die oberflächliche Frontallinie 5, die Laterallinien 7, die tiefen Rückenlinien und die Spirallinie 3, die sich über den ganzen Körper erstrecken und sich auch im Fuß 9 fortsetzen, wie in Fig. 1 gezeigt ist, Besonders hervorzuheben ist die Muskelfaszien- Spirallinie 3, die am Fuß im Bereich des vorderen unteren Sprunggelenks und der Fußwurzel verläuft und die anderen Muskel-Faszienzüge 1, 5 und 7 umfasst und bündelt.

Die Muskelfaszien-Spirallinie 3 verläuft wie eine Doppelhelix um den menschlichen Körper. Sie verbindet die Schädelseiten über den oberen Rücken jeweils mit der gegenüberliegenden Schulter, verläuft dann um die Rippen herum, kreuzt die andere Spirallinie der Doppelhelix auf der Vorderseite des Bauches und verläuft über Ober- und Unterschenkel hinunter zur Innenseite des Längsgewölbes des Fußes, dann unter dem Fuß 9 hindurch auf die Außenseite des Fußes 9 und hinter dem Außenknöchel aufwärts zur Außenseite des Unterschenkels und zum Sitzbein und von dort über den Rücken zurück zum Schädel (vgl. Thomas B. Meiers, Anatomy Trains - Myofasziale Leitbahnen, Verlag Urban und Fischer, 2. Auflage, 2010).

An der Fußwurzel durchquert die Muskelfaszien-Spirallinie 3 einen Bereich, der bei jeglicher Belastung des Fußes angesprochen wird und somit das gesamte Körpergewicht auffängt und es auf den Vor- und Rückfuß verteilt.

Dementsprechend ist die erfindungsgemäße bandförmige Vorrichtung gemäß einer Ausführungsform so im Schuh angeordnet, dass sie der Muskelfaszien-Spirallinie 3 eines im Schuh positionierten Fußes folgt und dessen Muskeln und Sehnen aktiviert oder anregt. Vorzugsweise wird die Muskelfaszien-Spirallinie 3 durch einen gezielten Kontakt, der eine flächige Stimulation zur Folge hat, aktiviert.

Die bandförmige Vorrichtung ist so im Schuh angeordnet, dass sie den Schuhboden am Fußgewölbe in Höhe des vorderen unteren Sprunggelenkes quert. An dieser Position befindet sich die bandförmige Vorrichtung gegenüberliegend zur Muskelfaszien-Spirallinie auf der Unterseite des Fußes.

Abhängig von der Schafthöhe des Schuhs folgt die bandförmige Vorrichtung dem Verlauf der Muskelfaszien-Spirallinie am Fuß von der vorderen Kante des Unterschenkels außerhalb der vorderen Schienenbeinlcante in der Verlaufsrichtung des vorderen Schienbeinmuskels zur inneren Seite der Fußwurzel, von wo sich die Muskelfaszien-Spirallinie um den Kopf des Sprungbeins (Talus) und des Kahnbeins windet und schräg über die Fußsohle auf die Außenseite des Fußes fersenwärts der Basis des fünften Mittelfußknochens verläuft und dann hinter dem Außenknöchel zum Wadenbein hochsteigt und in Höhe des Wadenbeinköpfchens ausläuft. Gemäß einer weiteren Ausführungsform ist die bandförmige Vorrichtung so im Schuh angeordnet, dass sie den Schuhboden ausgehend von der Lage der Basis des fünften Mittelfußknochens des im Schuh angeordneten Fußes quert.

Gemäß einer weiteren Ausführungsform ist die bandförmige Vorrichtung zumindest teilweise unter dem Innenfutter des Schuhs angeordnet. Die bandförmige Vorrichtung kann beispielsweise am Material, insbesondere Obermaterial, des Schuhs auf dessen Innenseite befestigt, insbesondere daran angenäht, darauf aufgeklebt oder in dieses integriert sein. Beispielsweise kann im Material des Schuhs eine Aussparung, Tasche, Schlaufe vorgesehen werden, in der die bandförmige Vorrichtung zumindest teilweise oder vollständig angeordnet wird. Die bandförmige Vorrichtung kann jedoch auch in das Innenfutter des Schuhs integriert sein. Die bandförmige Vorrichtung kann auch in die Schnürung integriert sein, sodass durch Schließen der Schnürung ein Druck auf den Fuß im Bereich der Spirallinie durch die bandförmige Vorrichtung ausgeübt wird, die der Aktivierung des Fußes dient, Beispielsweise kann zumindest ein Ende der bandförmigen Vorrichtung eine(n) oder mehrere Ösen oder Haken aufweisen, durch die ein Schnürband geführt werden kann. Zumindest ein Ende der bandförmigen Vorrichtung könnte auch an einer Leiste bzw. einem Rand der Einstiegsöffnung des Schuhs befestigt sein, in der bzw. dem Ösen oder Haken der Schnürung oder ein Reißverschluss angeordnet sind.

Gemäß einer weiteren Ausführungsform ist die bandförmige Vorrichtung als Teil des Innenfutters des Schuhs ausgebildet. Die bandförmige Vorrichtung könnte beispielsweise in das Innenfutter integriert, insbesondere eingenäht oder eingewebt sein.

Gemäß einer weiteren Ausführungsform ist die bandförmige Vorrichtung als ein Polster oder als ein flaches Band aus einem Gewebe oder Textil ausgebildet, wie beispielsweise als ein Gurt, Bei einem Polster kann der Druck auf den Fuß im Bereich zumindest eines der in diesem Bereich verlaufenden Muskelfaszienzüge des Fußes, insbesondere der Muskelfaszien-Spirallinie allein durch die Gewichtskraft des Trägers des Schuhs hervorgerufen werden, ohne dass ein Spannen der bandförmigen Vorrichtung erforderlich ist. Bei einer mit einem Gurt gebildeten bandförmigen Vorrichtung kann zur Druckausübung ein Spannen mit Hilfe eines Klettverschlusses oder einer anderen Schließ- oder Fixierungsvorrichtung erforderlich sein.

Gemäß einer Ausführungsform weist die bandförmige Vorrichtung eine Breite zwischen 1 und 5cm, insbesondere eine Breite von 1cm, 2cm, 3cm, 4cm oder 5cm auf.

Gemäß einer anderen Ausführungsform ist die bandförmige Vorrichtung zumindest abschnittsweise aus einem elastischen Material ausgebildet, d.h. sie weist mindestens einen Abschnitt aus einem elastischen Material auf. Dieser Abschnitt kann eine Länge zwischen 1 und 5 cm, insbesondere eine Länge von 1cm, 2cm, 3cm, 4cm oder 5cm haben. Bei dieser Ausführungsform kann infolge eines auf die bandförmige Vorrichtung ausgeübten Zugs, der durch festlegen zumindest eines der Enden, wie beispielsweise durch einen Verschluss, am Schuh hervorgerufen wird, eine Druckausübung auf einen oder mehrere Muskelfaszienzüge im Fuß des Trägers erfolgen. Es ist denkbar Abschnitte der bandförmigen Vorrichtung oder die gesamte bandförmige Vorrichtung aus einem elastischen Material herzustellen.

Gemäß einer weiteren Ausführungsform weist der Schuh zumindest an der Außenseite des Schaftes zumindest eine Öffnung auf, durch welche ein Ende der bandförmigen Vorrichtung von der Innenseite des Schuhs zur Außenseite geführt werden kann.

Auf der Außenseite des Schuhs kann die bandförmige Vorrichtung mit dem anderen Ende der bandförmigen Vorrichtung, ggf, in Verbindung mit einer Schnürung des Schuhs, mittels einer Öse oder eines Knopfes oder Hakens am Schuh gespannt werden, sodass ein Druck auf einen oder mehrere Muskelfaszienzüge des Fußes des Trägers ausgeübt werden kann.

Gemäß einer weiteren Ausführungsform weist die Innensohle des Schuhs auf der Oberseite oder Unterseite eine die Innensohle querende Vertiefung auf, in der die bandförmige Vorrichtung angeordnet ist. Die Vertiefung dient der Führung der bandförmigen Vorrichtung im Bereich der Sohle. Gemäß einer Ausführungsform verläuft die Vertiefung von der Innenseite des Schuhs auf Höhe des Fußgewölbes zur Außenseite des Schuhs auf Höhe des Außenknöchels. Alternativ kann die Vertiefung auch vom Bereich des Fußgewölbes zur Basis des fünften Mittelfußknochens verlaufen. Anstelle einer Vertiefung könnte auch ein geschlossener Kanal vorgesehen sein, der in der Sohle ausgebildet ist,

Gemäß einer Ausführungsform erstreckt sich die bandförmige Vorrichtung zusammen mit der Vertiefung mit einem Winkel zwischen 20° und 90°, insbesondere 30° und 80°, 40° und 70°, 50° und 60 ° (Grad) zur Längsrichtung der Sohle.

Gemäß noch einer weiteren Ausführungsform ist die bandförmige Vorrichtung unter der Innensohle des Schuhs angeordnet,

Gemäß einer weiteren Ausführungsform sind im Innenfutter oder an der Innenseite des Schuhs unter dem Innenfutter zumindest ein Kanal oder eine oder mehrere Ösen oder Schlaufen ausgebildet, in dem bzw. in der die bandförmige Vorrichtung aufgenommen werden kann. Der Kanal oder die Öse(n) oder Schlaufe(n) haben die Funktion, die bandförmige Vorrichtung an der Innenseite des Schuhs im Bereich des dort verlaufenden entsprechenden Muskelfaszienzugs - bzw. oder der dort verlaufenden Muskelfaszienzüge zu halten.

Gemäß einer weiteren Ausführungsform weist die bandförmige Vorrichtung zwei Enden auf, von welchen sich zumindest eines in zwei Enden verzweigt. Es können sich auch die Enden auf beiden Seiten der Sohle in jeweils zwei oder sogar mehr Enden verzweigen. Die Verzweigung des zumindest einen Endes erfolgt entsprechend dem Verlauf des Muskelfaszienzugs auf der Innenseite oder Außenseite des Fußes und dessen Verzweigung, Insbesondere kann eines der Enden der sich verzweigenden bandförmigen Vorrichtung an der Innenseite des Schuhs vom Schuhboden in Richtung der Position des Innenknöchels und hinter dem Innenknöchel des Fußes zur Achillessehne verlaufen. Ein weiteres Ende der sich verzweigenden bandförmigen Vorrichtung auf der gegenüberliegenden Innenseite des Schuhs kann vom Schuhboden in Richtung des Außenknöchels und dann hinter dem Außenknöchel in Richtung des Wadenbeinköpfchens verlaufen. Die jeweiligen anderen Enden folgen vorzugweise dem oben beschriebenen Verlauf der anderen Züge der Muskelfaszien-Spirallinie.

Gemäß einer weiteren Ausführungsform weist die bandförmige Vorrichtung einen Verschluss auf, mit dem die beiden Enden, oder im Fall einer Verzweigung ggf. zusätzliche Enden, der bandförmigen Vorrichtung miteinander verbunden werden können. Damit kann die bandförmige Vorrichtung im Inneren des Schuhs oder auf der Außenseite über dem Fuß geschlossen werden und es kann ein Druck auf die Muskelfaszienzüge des Fußes entsprechend der Anordnung der bandförmigen Vorrichtung gegenüberliegend dem zumindest einen MuskelFaszienzug ausgeübt werden.

Gemäß einer weiteren Ausführungsform weist die bandförmige Vorrichtung zumindest an einem Ende eine oder mehrere Komponenten eines Klettverschlusses auf, sodass ein Ende der bandförmigen Vorrichtung, der die eine Komponente des Klettverschlusses aufweist, auf einen anderen Abschnitt desselben Endes der bandförmigen Vorrichtung mit der anderen Komponente des Klettverschlusses zurückgefaltet werden kann, nachdem das Ende durch eine oder mehrere Ösen am Schuh geführt wurde.

Es sind jedoch auch andere Arten von Verschlüssen denkbar, um eines oder beide Enden der bandförmigen Vorrichtung am Schuh zu fixieren oder mit einander zu verbinden, wie z.B. ein Klemmverschluss oder eine Schnalle,

Gemäß noch einer weiteren Ausführungsform weist die bandförmige Vorrichtung im Inneren einen Kanal zur Aufnahme eines Fluids auf, das mit einem Druck beaufschlagt werden kann. Bei dem Fluid kann es sich beispielsweise um ein Gas, eine Flüssigkeit oder ein Memory-Material handeln.

Gemäß einer weiteren Ausführungsform kann der Schuh zusätzlich zumindest eine weitere bandförmige Vorrichtung aufweisen, die von der anderen bandförmigen Vorrichtung getrennt ist oder einen Teil derselben bildet. Gemäß noch einer weiteren Ausführungsform kann die weitere bandförmige Vorrichtung als ein Polster ausgebildet sein, das in das Innenfutter des Schuhs integriert ist, während die andere bandförmige Vorrichtung z.B. durch einen Gurt oder ein textiles Gewebe gebildet ist. Eine derartige Kombination von Materialien ist auch im Fall einer bandförmigen Vorrichtung mit einem oder zwei verzweigten Enden denkbar, wobei die verzweigten Enden aus unterschiedlichen Materialien, insbesondere als ein Polster und als ein Gurt oder ein textiles Gewebe, gebildet sind.

Die weitere bandförmige Vorrichtung kann entsprechend der Lage weiterer Muskelfaszienzüge im Schuh angeordnet sein, die auf der Vorderseite des Außenknöchels und/oder auf der Innenseite der Ferse im Bereich der Achillessehne verlaufen. Dadurch können zusätzliche Haltefunktionen, hauptsächlich passiver, aber auch aktiver Art durch Einfluss auf z.B. die Muskelfaszienkette der tiefen Frontallinie erreicht werden. Vorzugsweise sollte die bandförmige Vorrichtung, die dem Verlauf der Muskelfaszien-Spirallinie entspricht, immer vorhanden sein.

Gemäß einer Ausführungsform ist zumindest ein Ende der bandförmigen Vorrichtung mit der Schnürung verbunden. Ist die bandförmige Vorrichtung in die Schnürung integriert, wird durch Schließen der Schnürung ein leichter Druck auf die Haut im Bereich der Muskelfaszienspirallinie ausgeübt, die deren Aktivierung anregt.

Gemäß einer weiteren Ausführungsform ist zumindest ein Ende der bandförmigen Vorrichtung am Schuh, insbesondere an der Innenseite festgelegt. Gemäß dieser Ausführungsform ist vorgesehen, lediglich auf ein Ende der bandförmigen Vorrichtung, das nicht festgelegt ist, einen Zug auszuüben und dieses Ende am Schuh insbesondere auf der Außenseite des Schuhs oder in Verbindung mit der Schnürung festzulegen, um die bandförmige Vorrichtung zu spannen und einen Druck auf Muskelfaszienzüge am Fuß des Trägers auszuüben.

Erfindungsgemäß wird des Weiteren eine Schuhinnensohle bereitgestellt, welche eine bandförmige Vorrichtung aufweist, welche mit der Sohle verbunden ist und sich unter einem Winkel zur Längsrichtung der Sohle erstreckt.

Durch die in die Schuhinnensohle integrierte oder damit verbundene bandförmige Vorrichtung werden im Bereich der bandförmigen Vorrichtung liegende Abschnitte des Fußes durch Ausübung eines Drucks stimuliert und damit die Funktionen des Fußes und des Unterschenkels, die in einem herkömmlichen Schuh beeinträchtigt werden oder zum Erliegen kommen, über die Tragezeit der erfindungsgemäßen Schuhinnensohle aktiviert werden. Der ausgeübte Druck kann abhängig von der Ausführung der bandförmigen Vorrichtung für den Träger der Schuhinnensohle kaum wahrnehmbar oder deutlich spürbar sein. Auch kann der durch die bandförmige Vorrichtung ausgeübte Druck in verschiedenen Bereichen der bandförmigen Vorrichtung hinsichtlich der Stärke variieren.

Gemäß einer bevorzugten Ausführungsform ist die bandförmige Vorrichtung so an der Schuhinnensohle angeordnet oder in diese integriert, dass sie auf Muskelfaszienzüge des Fußes einen Druck ausübt. Die bandförmige Vorrichtung ist so an der Schuhinnensohle angeordnet, dass sie entlang einer oder mehrerer Muskelfaszienzüge des Fußes verläuft.

Mit einer derartigen Innensohle, die in einen Schuh oder Stiefel eingelegt werden kann, können bestimmte Muskelfaszienzüge im Fuß, die von der bandförmigen Vorrichtung berührt werden, stimuliert werden, Insbesondere wird mit der Schuhinnensohle ein Druck auf einen oder mehrere Muskelfaszienzüge im Fuß ausgeübt. Der Druck kann beispielsweise durch das Körpergewicht beim Tragen eines Schuhs mit der erfindungsgemäßen Schuhinnensohle erzeugt werden.

Die bandförmige Vorrichtung ist so an der Schuhinnensohle angeordnet oder in diese integriert, dass sie den Schuhboden am Fußgewölbe in Höhe des vorderen unteren Sprunggelenkes quert. An dieser Position befindet sich die bandförmige Vorrichtung gegenüberliegend zur Muskelfaszien-Spirallinie auf der Unterseite des Fußes.

Gemäß einer Ausführungsform folgt die bandförmige Vorrichtung dem Verlauf der Muskelfaszien-Spirallinie am Fuß von der vorderen Kante des Unterschenkels außerhalb der vorderen Schienenbeinkante in der Verlaufsrichtung des vorderen Schienbeinmuskels zur inneren Seite der Fußwurzel, von wo sich die Muskelfaszien-Spirallinie um den Kopf des Sprungbeins (Talus) und des Kahnbeins windet und schräg über die Fußsohle auf die Außenseite des Fußes fersenwärts der Basis des fünften Mittelfußknochens verläuft und dann hinter dem Außenknöchel zum Wadenbein hochsteigt und in Höhe des Wadenbeinköpfchens ausläuft.

Gemäß einer weiteren Ausführungsform ist die bandförmige Vorrichtung so an der Schuhinnensohle angeordnet oder in diese integriert, dass sie den Schuhboden ausgehend von der Lage der Basis des fünften Mittelfußknochens des im Schuh angeordneten Fußes quert.

Gemäß einer Ausführungsform erstreckt sich die bandförmige Vorrichtung oder einer oder mehrere Abschnitte derselben mit einem Winkel zwischen 20° und 90°, insbesondere 30° und 80°, 40° und 70°, 50° und 60 ° oder 60° und 70° (Grad) zur Längsrichtung der Sohle.

Gemäß einer Ausführungsform ist die bandförmige Vorrichtung in die Schuhinnensohle integriert oder auf der Ober-oder Unterseite der Schuhinnensohle angeordnet. Insbesondere kann die bandförmige Vorrichtung fest mit der Schuhinnensohle verbunden sein oder aus einem Stück mit der Schuhinnensohle hergestellt sein.

Gemäß einer weiteren Ausführungsform weist die Schuhinnensohle auf der Oberseite oder Unterseite eine die Schuhinnensohle querende Vertiefung auf, in der die bandförmige Vorrichtung angeordnet ist.

Gemäß einer weiteren Ausführungsform kann sich die bandförmige Vorrichtung von einer oder von beiden Seiten der Schuhinnensohle als ein Fortsatz derselben erstrecken. Die bandförmige Vorrichtung erstreckt sich in Richtung eines oder mehrerer Muskelfaszienzüge im Fuß des Trägers, wie beispielsweise entlang der Muskelfaszien-Spirallinie und mit einem Winkel zur Längsrichtung der Sohle, wie oben beschrieben wurde. Die in die Schuhinnensohle integrierte bandförmige Vorrichtung kann sich beispielsweise über eine Strecke von 1cm, 2cm, 3cm, 4cm, 5cm, 6cm, 7cm, 8cm, 9cm, 10cm, 15cm, 20cm oder mehr auf einer oder auf beiden Seiten der Schuhinnensohle erstrecken. Auch können sich von der Schuhinnensohle auf einer Seite oder auf beiden Seiten Enden einer bandförmigen Vorrichtung erstrecken, die eine Länge aufweisen, die so gewählt ist, dass sie miteinander über dem Fuß verbunden werden können oder zumindest aus dem Schaft des Schuhs ragen, um an diesem festgelegt zu werden.

Die von der Schuhinnensohle ausgehenden Abschnitte der bandförmigen Vorrichtung erstrecken sich bei der in einem Schuh angeordneten Schuhinnensohle von der Sohle im Wesentlichen senkrecht nach oben in Richtung des Schaftrandes. Es ist jedoch auch denkbar, dass einer oder beide Abschnitte unter einem Winkel zwischen 0° und 45° im Verhältnis zur Senkrechten auf die Sohle des Schuhs nach oben verlaufen, um besser dem Verlauf der Muskelfaszienzüge am Fuß zu entsprechen. Der Winkel kann insbesondere zwischen 20° und 70°, zwischen 30° und 60° oder zwischen 35° und 55° liegen.

Die in die Schuhinnensohle integrierte oder damit verbundene bandförmige Vorrichtung kann auch eine oder mehrere Eigenschaften, insbesondere hinsichtlich der Gestaltung und Ausführung der bandförmigen Vorrichtung, aufweisen, die oben im Zusammenhang mit dem Schuh beschrieben wurden, der eine bandförmige Vorrichtung aufweist.

Erfindungsgemäß wird weiter ein Strumpf bereitgestellt, welcher eine bandförmige Vorrichtung aufweist, die sich an einer Innenseite des Strumpfs vom oberen Ende zum Fußteil des Strumpfs und auf der gegenüberliegenden Innenseite des Strumpfs vom Fußteil zum oberen Ende erstreckt, wobei die bandförmige Vorrichtung eingerichtet ist, auf einen im Strumpf angeordneten Fuß einen Druck auszuüben.

Die bandförmige Vorrichtung ist so im Strumpf angeordnet, dass sie auf zumindest einen Muskelfaszienzug des Fußes einen Druck ausübt. Die bandförmige Vorrichtung ist so im Strumpf angeordnet oder in diesen integriert sein, dass sie entlang eines oder mehrerer Muskelfaszienzüge des Fußes verläuft.

Durch die in den Strumpf integrierte oder damit verbundene bandförmige Vorrichtung können im Bereich der bandförmigen Vorrichtung liegende Abschnitte des Fußes durch Ausübung eines Drucks stimuliert und damit die Funktionen des Fußes und des Unterschenkels, die in einem herkömmlichen Strumpf beeinträchtigt werden oder zum Erliegen kommen, über die Tragezeit des erfindungsgemäßen Strumpfs aktiviert werden. Der ausgeübte Druck kann abhängig von der Ausführung der bandförmigen Vorrichtung für den Träger des Strumpfs kaum wahrnehmbar oder deutlich spürbar sein. Auch kann der durch die bandförmige Vorrichtung ausgeübte Druck in verschiedenen Bereichen der bandförmigen Vorrichtung hinsichtlich der Stärke variieren. Verläuft die bandförmige Vorrichtung beispielsweise im Strumpf über den Bereich der Achillessehne, kann die bandförmige Vorrichtung an dieser Stelle dünner gestaltet oder ausgespart sein, um Reibungen am Fuß zu vermeiden.

Gemäß einer weiteren Ausführungsform weist die bandförmige Vorrichtung einen oder mehrere Abschnitte auf, die miteinander verbunden oder getrennt sein können. Ein Abschnitt der bandförmigen Vorrichtung kann sich unter einem Winkel zur Längsrichtung des Fußbereichs auf der Unterseite des Strumpfs erstrecken. Die bandförmige Vorrichtung ist so im Strumpf angeordnet ist, dass sie den Fußteil des Strumpfs am Fußgewölbe quert. Gemäß noch einer anderen Ausführungsform ist die bandförmige Vorrichtung so im Strumpf angeordnet, dass sie den Fußteil des Strumpfs ausgehend von der Lage der Basis des fünften Mittelfußknochens des Fußes im Strumpf quert. Die bandförmige Vorrichtung folgt dem Verlauf der Muskelfaszien-Spirallinie am Fuß von der vorderen Kante des Unterschenkels außerhalb der vorderen Schienenbeinkante in der Verlaufsrichtung des vorderen Schienbeinmuskels zur inneren Seite der Fußwurzel, von wo sich die Muskelfaszien-Spirallinie um den Kopf des Sprungbeins (Talus) und des Kahnbeins windet und schräg über die Fußsohle auf die Außenseite des Fußes fersenwärts der Basis des fünften Mittelfußknochens verläuft und dann hinter dem Außenknöchel zum Wadenbein hochsteigt und in Höhe des Wadenbeinköpfchens ausläuft.

Gemäß einer Ausführungsform erstreckt sich die bandförmige Vorrichtung mit einem Winkel zwischen 20° und 90°, insbesondere 30° und 80°, 40° und 70°, 50° und 60 ° (Grad) zur Längsrichtung des Fußbereichs des Strumpfs an der Unterseite desselben,

Die bandförmige Vorrichtung kann in den Strumpf integriert sein. Beispielsweise kann die bandförmige Vorrichtung auf der Innenseite oder auf der Außenseite des Strumpfs aufgebracht oder in einer in den Strumpf integrierten Tasche angeordnet sein. Die bandförmige Vorrichtung oder Abschnitte derselben könnten auch in das Material des Strumpfs eingewoben sein.

An den seitlichen Bereichen des Strumpfs erstreckt sich die bandförmige Vorrichtung im Wesentlichen senkrecht nach oben in Richtung des oberen Randes des Strumpfs. Die bandförmige Vorrichtung kann sich bis zum oberen Rand des Strumpfs erstrecken. Einer oder beide Abschnitte der sich auf den Seiten des Strumpfs aufwärts erstreckenden Abschnitte der bandförmigen Vorrichtung können sich auch unter einem Winkel insbesondere zwischen 20° und 70°, zwischen 30° und 60° oder zwischen 35° und 55° im Verhältnis zur Senkrechten auf die Unterseite bzw. untere Fußfläche des Strumpfs erstrecken.

Gemäß einer weiteren Ausführungsform ist die bandförmige Vorrichtung so in dem Strumpf angeordnet oder in diese integriert, dass sie die Unterseite des Strumpfs am Fußgewölbe, insbesondere in Höhe des vorderen unteren Sprunggelenkes quert. An dieser Position befindet sich die bandförmige Vorrichtung gegenüberliegend zur Muskelfaszien-Spirallinie auf der Unterseite des Fußes. Der Strumpf kann aus einem textilen Material, aus Baumwolle, Wolle, Kunststoffmaterial oder einer Kombination daraus hergestellt sein,

Die in dem Strumpf angeordnete oder in diese integrierte bandförmige Vorrichtung kann auch eine oder mehrere Eigenschaften, insbesondere hinsichtlich der Gestaltung und Ausführung der bandförmigen Vorrichtung, aufweisen, die oben im Zusammenhang mit dem Schuh oder der Schuhinnensohle beschrieben wurden, die eine bandförmige Vorrichtung aufweisen.

Weitere Merkmale, Eigenschaften und Vorteile der Erfindung ergeben sich aus der nachfolgenden Beschreibung der Erfindung anhand eines bespielhaften Ausführungsbeispiels und der angefügten Zeichnung. In der Zeichnung zeigt
- Fig. 1: den Verlauf der Muskelfaszien-Linien am menschlichen Fuß;
- Fig. 2a, b, c: den Verlauf der Muskelfaszien-Spirallinie am menschlichen Fuß in einer Ansicht von unten (Fig. 2a), in einer Ansicht von der Außenseite (Fig. 2b), und in einer Ansicht von der Innenseite (Fig. 2c) entsprechend dem Verlauf der bandförmigen Vorrichtung in einem Schuh;
- Fig. 3a, b: Ansichten der Innenseite eines Schuhs auf die Schuhaußenseite (Fig. 3a) bzw. Schuhinnenseite (Fig. 3b); und
- Fig. 4a, b, c: Ansichten eines Strumpfs gemäß einer Ausführungsform der Erfindung von unten (Fig. 4a), in einer Ansicht von der Außenseite (Fig, 4b), und in einer Ansicht von der Innenseite (Fig. 4c) gemäß einer Ausführungsform der Erfindung.

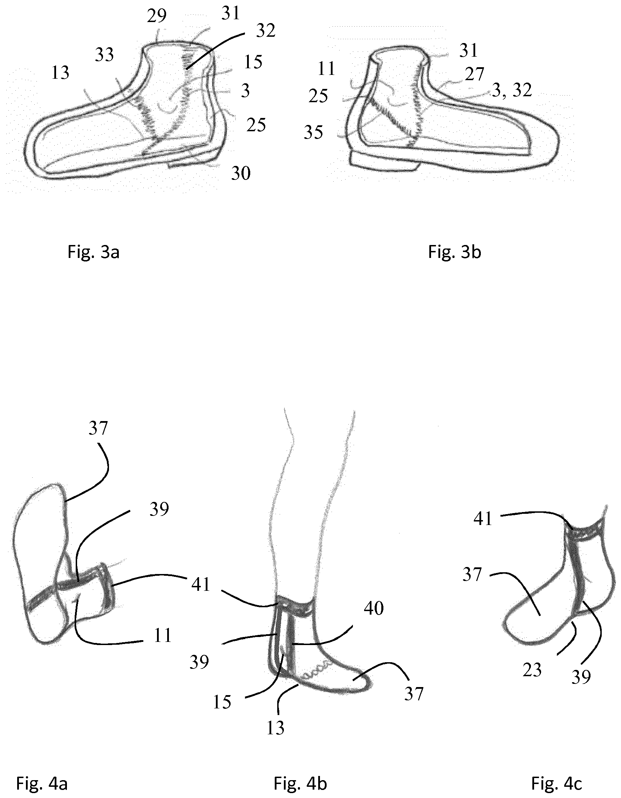

Eine Ausführungsform der Erfindung wird im Folgenden anhand der Figuren beschrieben, In Fig. 2a ist ein Fuß von schräg unten gezeigt, wobei der Innenknöchel 11 angedeutet ist. Der Verlauf der bandförmigen Vorrichtung folgt in einem Schuh 27, wie er in den Figuren 3a, 3b gezeigt ist, zumindest teilweise der Muskelfaszien-Spirallinie 3, die von der Vorderseite der Schienbeinkante um den Kopf des Sprungbeins und des Kahnbeins unter das Fußgewölbe 23 zur Außenseite des Fußes 9 hinter der Basis 13 des fünften Mittelfußknochens verläuft.

In Figur 2b ist der Fuß 9 von außen dargestellt, Die Muskelfaszien-Spirallinie 3 erscheint, von der Fußsohle her kommend, auf der Fußaußenseite hinter der Basis 13 des fünften Mittelfußknochens und verläuft in Richtung der Achillessehne 25 und hinter dem Außenknöchel 15 in Richtung des Wadenbeinköpfchens 17 und nähert sich dabei dem innen verlaufenden Zügel der Muskelfaszien-Spirallinie 3, Die bandförmige Vorrichtung 32 im Schuh 27 folgt zumindest teilweise dem Verlauf der Muskelfaszien-Spirallinie 3.

Gemäß einer Ausführungsform ist eine Erweiterung 33 der bandförmigen Vorrichtung 32 auf der Fußaußenseite des Schuhs 27 vorgesehen, die an der Stelle beginnt, an der die bandförmige Vorrichtung entsprechend der Muskelfaszien-Spirallinie 3 von der Fußsohle kommend hinter der Basis des fünften Mittelfußknochens 13 an der Fußaußenseite erscheint, Die Erweiterung 33 der bandförmigen Vorrichtung kann mit der bandförmigen Vorrichtung 32 verbunden sein oder von dieser getrennt sein. Sie verläuft entsprechend der Muskelfaszien-Linie 19 vor dem Außenknöchel 15 hinauf zum Unterschenkel und in etwa parallel zum Verlauf des Zügels der bandförmigen Vorrichtung 32 entsprechend der Muskelfaszien-Spirallinie 3.

In der Figur 2c ist der Fuß 9 in der Ansicht von der Innenseite dargestellt. Die Muskelfaszien-Spirallinie 3 verläuft von der vorderen Schienbeinkante kommend vor dem Innenknöchel 11 an der Stelle, an der das Fußgewölbe 23 ausgeprägt ist, unter die Fußsohle. Darüber befindet sich das Kahnbein und der Kopf des Sprungbeins. Die bandförmige Vorrichtung 32 im Schuh 27 folgt zumindest teilweise dem Verlauf der Muskelfaszien-Spirallinie 3 auf dieser Seite des Fußes.

Gemäß einer weiteren Ausführungsform kann die bandförmige Vorrichtung 32 um eine weitere bandförmige Vorrichtung 35 an der Schuhinnenseite entsprechend einer Muslcelfaszien-Linie 18 erweitert sein. Diese zusätzliche bandförmige Vorrichtung 35 setzt entsprechend der Muskelfaszien-Spirallinie 3 in Höhe des Fußgewölbes 23 bzw. Kahnbeins an der bandförmigen Vorrichtung 32 an und verläuft auf der Innenseite der Ferse nach oben. Sie kann gemäß einer Ausführungsform fortgesetzt bzw. verlängert sein und oberhalb des Fersenbeins den Bereich der Achillessehne 25 kreuzen und auf der gegenüberliegenden Außenseite des Fußes den äußeren Zügel der bandförmigen Vorrichtung 32 entsprechend der dort verlaufenden Muskelfaszien-Spirallinie 3 treffen (Diese Fortsetzung ist in Fig. 3a nicht dargestellt), Im Bereich der Kreuzung der Achillessehne 25 kann sich die Dicke und/oder auch die Härte der bandförmigen Vorrichtung 35 verändern, insbesondere reduziert oder ausgespart sein, um Reibeeffekte an der Achillessehne 25 zu vermeiden.

In den Figuren 3a, b ist die Erfindung an einem aufgeschnittenen Schuh gemäß einer Ausführungsform demonstriert. In Figur 3a ist der Schuh 27 aufgeschnitten dargestellt, wobei die Kleinzehenseite im Schuh 27 entsprechend der Position der Fuß-Außenseite im Schuh 27 zu erkennen ist. Das Innenfutter 29 des Schuhs ist ebenfalls aufgeschnitten dargestellt. An einer Verdickung 31 des Innenfutters am oberen Schaftrand erkennt man den Verlauf der prominenten erfindungsgemäßen bandförmigen Vorrichtung 32. Diese verläuft entsprechend der Muskelfaszien-Spirallinie 3 hinter dem angedeuteten Außenknöchel 15 des Fußes im Schuh 27 zunächst im Wesentlichen senkrecht nach unten. Die bandförmige Vorrichtung 32 quert den Schuhboden 30 bzw. unterfährt die Fußsohle beginnend auf der Kleinzehenseite des Schuhs 27 bzw. Außenseite des Fußes etwa hinter der Position der Basis 13 des angedeuteten fünften Mittelfußknochens des Fußes im Schuh, Eine zusätzliche bandförmige Vorrichtung 33 entsprechend der in Fig, 2b gezeigten Muskelfaszien-Linie 19 kann ausgehend von der ursprünglichen bandförmigen Vorrichtung 32 auf der Kleinzehenseite der Innenseite des Schuhs 27 vor der Position des Außenknöchels 15 im Schuh in Höhe des Ristes verlaufen.

Die in der Fig. 3b aufgeschnittene Darstellung des Schuhs 27 zeigt eine Sicht auf die Großzehenseite der Schuhinnenseite, Die erfindungsgemäße bandförmige Vorrichtung 32 verläuft entsprechend der Muskelfaszien-Spirallinie 3 vor der Position des Innenknöchels 11 im Schuh 27 im Wesentlichen senkrecht vom Schaftrand und quert den Schuhboden bzw. unterfährt die Fußsohle im Bereich des Sprungbeinkopfes und des Kahnbeins etwa an der Stelle, an der sich das Längsgewölbe 23 des Fußes 9 befindet. An der Verdickung 31 am oberen Rand des Schaftes ist erkennbar, dass die bandförmige Vorrichtung 32 von oben, lateral der Schienbeinkante in den Schuh 27 hinein verläuft. Die bandförmige Vorrichtung 32 kann auf der Großzehenseite des Schuhs 27 durch eine zusätzliche bandförmige Vorrichtung 35 erweitert werden, die am Umschlag der bandförmigen Vorrichtung 32 ausgehend von der bandförmigen Vorrichtung 32 an der Position des Fußgewölbes des Fußes im Schuh fersenwärts zur Position der Achillessehne 25 oberhalb des Fersenbeins verlaufen. Die zusätzliche bandförmige Vorrichtung 35 kann im Bereich der Achillessehne 25 ausgedünnt oder ausgespart sein und ihr Verlauf kann sich im Innenfutter 29 auf der Außenseite des Schuhs 27 fortsetzen. Die zusätzlichen bandförmigen Vorrichtungen 33, 35 können aus demselben Material wie die entsprechenden Abschnitte der bandförmigen Vorrichtung 3, von der sie ausgehen, gebildet sein, oder aus einem anderen Material. Beispielsweise können die zusätzlichen bandförmigen Vorrichtungen 33, 35 durch eine Polsterung gebildet sein, während die bandförmige Vorrichtung 3 aus einem Gurtmaterial gebildet ist oder umgekehrt.

In den Figuren 4A bis 4C ist ein Strumpf 37 gemäß einer Ausführungsform der Erfindung in verschiedenen Ansichten gezeigt. In Fig. 4a ist der Strumpf 37 von schräg unten gezeigt. Der Verlauf der bandförmigen Vorrichtung 39 folgt am Strumpf 37 zumindest teilweise der Muskelfaszien-Spirallinie 3, die von der Vorderseite der Schienbeinkante um den Kopf des Sprungbeins und des Kahnbeins unter das Fußgewölbe 23 zur Außenseite des Fußes 9 hinter der Basis 13 des fünften Mittelfußknochens verläuft, wie oben im Zusammenhang mit dem Schuh 27 angegeben wurde,

In Figur 4b ist der Strumpf 37 von außen dargestellt. Wie oben beschrieben, erscheint die Muskelfaszien-Spirallinie 3, von der Fußsohle her kommend, an der Fußaußenseite hinter der Basis 13 des fünften Mittelfußknochens und verläuft in Richtung der Achillessehne und hinter dem Außenknöchel 15 in Richtung des Wadenbeinköpfchens 17 (siehe Fig. 1). Die bandförmige Vorrichtung 39 im Strumpf 37 folgt zumindest teilweise, insbesondere bis zum Bund 41 des Strumpfes 37, dem Verlauf der Muskelfaszien-Spirallinie 3.

Gemäß einer Ausführungsform ist wie beim Schuh eine Erweiterung 40 der bandförmigen Vorrichtung 39 auf der Fußaußenseite des Strumpfs 37 vorgesehen, die an der Stelle beginnt, an der die bandförmige Vorrichtung 39 entsprechend der Muskelfaszien-Spirallinie 3 von der Fußsohle kommend hinter der Basis des fünften Mittelfußknochens 13 an der Fußaußenseite erscheint. Die Erweiterung 40 der bandförmigen Vorrichtung kann mit der bandförmigen Vorrichtung 39 verbunden sein oder von dieser getrennt sein. Sie verläuft entsprechend der Muskelfaszien-Linie 19 vor dem Außenknöchel 15 hinauf zum Unterschenkel und in etwa parallel zum Verlauf des Zügels der bandförmigen Vorrichtung 39 entsprechend der Muskelfaszien-Spirallinie 3 (Siehe Fig. 2b).

In der Figur 4c ist der Strumpf 37 in der Ansicht von der Innenseite dargestellt, Wie bereits oben beschrieben und in Fig. 2c gezeigt, verläuft die Muskelfaszien-Spirallinie 3 von der vorderen Schienbeinkante kommend vor dem Innenknöchel 11 an der Stelle, an der das Fußgewölbe 23 ausgeprägt ist, unter die Fußsohle. Darüber befinden sich das Kahnbein und der Kopf des Sprungbeins, Die bandförmige Vorrichtung 39 im Strumpf 37 folgt zumindest teilweise dem Verlauf der Muskelfaszien-Spirallinie 3 auf dieser Seite des Fußes 9.

Gemäß einer weiteren Ausführungsform kann die bandförmige Vorrichtung 39 um eine weitere (nicht gezeigte) bandförmige Vorrichtung an der Innenseite des Strumpfs 37 entsprechend der Muskelfaszien-Linie 18 erweitert sein. Diese zusätzliche bandförmige Vorrichtung setzt entsprechend der Muskelfaszien-Spirallinie 3 in Höhe des Fußgewölbes 23 bzw. Kahnbeins an der bandförmigen Vorrichtung 39 an und verläuft auf der Innenseite der Ferse nach oben, kreuzt oberhalb des Fersenbeins den Bereich der Achillessehne 25 und trifft auf der gegenüberliegenden Außenseite des Fußes den äußeren Zügel der bandförmigen Vorrichtung 39 entsprechend der dort verlaufenden Muskelfaszien-Spirallinie 3. Im Bereich der Kreuzung der Achillessehne 25 kann sich die Dicke und/oder auch die Härte der bandförmigen Vorrichtung 39 im Strumpf verändern, insbesondere reduziert oder ausgespart sein, um Reibeeffekte an der Achillessehne 25 zu vermeiden.

### Bezugszeichen:

- 1, 5, 7: Muskel-Faszienzüge
- 3: Muskelfaszien-Spirallinie
- 9: Fuß
- 11: Innenknöchel
- 13: Basis des fünften Mittelfußknochens
- 15: Außenknöchel
- 17: Wadenbeinköpfchen
- 19: Muskelfaszien-Linie
- 21: Kahnbein
- 23: Fußgewölbe
- 25: Bereich der Achillessehne
- 27: Schuh
- 29: Innenfutter
- 30: Schuhboden
- 31: Verdickung
- 32: Bandförmige Vorrichtung
- 33: Zusätzliche bandförmige Vorrichtung
- 35: Zusätzliche bandförmige Vorrichtung
- 37: Strumpf
- 39: Bandförmige Vorrichtung am Strumpf
- 40: Erweiterung der bandförmigen Vorrichtung am Strumpf
- 41: Strumpfbund

## Patentansprüche

1. Schuh, welcher eine bandförmige Vorrichtung (32) aufweist, die so an einer Innenseite des Schuhs (27) befestigt ist, dass sie in der Richtung vom oberen Schaftende des Schuhs zum Schuhboden entlang zumindest eines von der Innenseite des Längsgewölbes des Fußes in Richtung des Unterschenkels verlaufenden Muskelfaszienzugs (1, 3, 5, 7) eines im Schuh angeordneten Fußes (9) verläuft, um auf den zumindest einen Muskelfaszienzug (1, 3, 5, 7) einen Druck auszuüben, wobei die bandförmige Vorrichtung den Schuhboden entsprechend dem Muskelfaszienzug (1, 3, 5, 7) am Fußgewölbe in Höhe des vorderen unteren Sprunggelenks quert.

2. Schuh gemäß Anspruch 1, wobei die bandförmige Vorrichtung (32) so im Schuh (27) angeordnet ist, dass sie den Schuhboden ausgehend von der Lage der Basis (13) des fünften Mittelfußknochens des Fußes (9) im Schuh (27) quert.

3. Schuh gemäß einem der Ansprüche 1 oder 2, wobei die bandförmige Vorrichtung (32) zumindest teilweise unter dem Innenfutter (29) des Schuhs (27) angeordnet ist oder als Teil des Innenfutters (29) des Schuhs (27) ausgebildet ist.

4. Schuh gemäß einem der Ansprüche 1 bis 3, wobei die bandförmige Vorrichtung (32) als ein Polster oder als ein flaches Band aus einem Gewebe oder Textil ausgebildet ist.

5. Schuh gemäß einem der Ansprüche 1 bis 4, wobei der Schuh (27) an der Außenseite des Schaftes zumindest eine Öffnung aufweist, durch welche ein Ende der bandförmigen Vorrichtung (32) von der Innenseite des Schuhs (27) zur Außenseite geführt werden kann.

6. Schuh gemäß einem der Ansprüche 1 bis 5, wobei im Innenfutter (29) oder an der Innenseite des Schuhs (27) unter dem Innenfutter (29) zumindest ein Kanal oder eine oder mehrere Ösen oder Schlaufen ausgebildet sind, in dem bzw. in der die bandförmige Vorrichtung (32) aufgenommen werden kann.

7. Schuh gemäß einem der Ansprüche 1 bis 6, wobei die bandförmige Vorrichtung (32) zwei Enden aufweist, von welchen sich zumindest eines in zwei Enden verzweigt, wobei die bandförmige Vorrichtung (32) einen Verschluss aufweist, mit dem die beiden Enden der bandförmigen Vorrichtung (32) miteinander verbunden werden können.

8. Schuh gemäß einem der Ansprüche 1 bis 7, wobei zumindest ein Ende der bandförmigen Vorrichtung (32) mit der Schnürung verbunden.

9. Schuhinnensohle, welche eine bandförmige Vorrichtung (32) aufweist, welche mit der Sohle verbunden ist und sich unter einem Winkel zur Längsrichtung der Sohle erstreckt, wobei die bandförmige Vorrichtung (32) eingerichtet ist, dass sie sich, wenn sie in einem Schuh (27) angeordnet ist, in einer Richtung vom oberen Schaftende zum Schuhboden erstreckt und dabei in der Richtung vom oberen Schaftende zum Schuhboden entlang zumindest eines von der Innenseite des Längsgewölbes des Fußes in Richtung des Unterschenkels verlaufenden Muskelfaszienzugs (1, 3, 5, 7) eines im Schuh angeordneten Fußes (9) verläuft, wobei die bandförmige Vorrichtung (32) so an der Schuhinnensohle angeordnet oder in diese integriert ist, dass sie den Schuhboden am Fußgewölbe in Höhe des vorderen unteren Sprunggelenks quert, um auf den zumindest einen Muskelfaszienzug (1, 3, 5, 7) des im Schuh (27) angeordneten Fußes (9) einen Druck auszuüben.

10. Schuhinnensohle gemäß Anspruch 9, wobei sich die bandförmige Vorrichtung (32) oder einer oder mehrere Abschnitte derselben mit einem Winkel zwischen 20° und 90°, insbesondere 30° und 80°, 40° und 70°, 50° und 60 ° oder 60° und 70° zur Längsrichtung der Innensohle erstreckt.

11. Strumpf, welcher eine bandförmige Vorrichtung (39) aufweist, die sich derart an einer Innenseite des Strumpfs (37) in einer Richtung vom oberen Ende zum Fußteil des Strumpfs (37) erstreckt, dass sie in der Richtung vom oberen Ende zum Fußteil des Strumpfs entlang zumindest eines von der Innenseite des Längsgewölbes des Fußes in Richtung des Unterschenkels verlaufenden Muskelfaszienzugs (1, 3, 5, 7) eines im Strumpf angeordneten Fußes (9) verläuft und die Unterseite des Fußes am Fußgewölbe in Höhe des vorderen unteren Sprunggelenks quert, um auf den zumindest einen Muskelfaszienzug (1, 3, 5, 7) des im Strumpf (37) angeordneten Fußes (9) einen Druck auszuüben.

12. Strumpf gemäß Anspruch 11, wobei die bandförmige Vorrichtung (39) so im Strumpf (37) angeordnet ist, dass sie das Fußteil des Strumpfs (37) am Fußgewölbe quert.

13. Strumpf gemäß einem der Ansprüche 11 oder 12, wobei die bandförmige Vorrichtung (39) so im Strumpf (37) angeordnet ist, dass sie das Fußteil ausgehend von der Lage der Basis (13) des fünften Mittelfußknochens des Fußes (9) im Strumpf (37) quert.

## Claims

1. Shoe which has a band-like device (32) attached to an inner side of the shoe (27) to extend in the direction from the upper shaft end of the shoe to the shoe base along at least one muscle fascia (1, 3, 5, 7) of a foot (9) arranged in the shoe, extending from the inner side of the longitudinal arch of the foot toward the lower leg, in order to exert pressure on the at least one muscle fascia (1, 3, 5, 7), the band-like device (3) crossing the shoe base according to the muscle fascia (1, 3, 5, 7) at the arch of the foot at the level of the anterior lower ankle joint.

2. Shoe according to Claim 1, wherein the band-like device (32) is arranged in the shoe (27) such that it crosses the shoe base starting from the location of the base (13) of the fifth metatarsal of the foot (9) in the shoe (27).

3. Shoe according to one of Claims 1 to 2, wherein the band-like device (32) is arranged at least partially under the inner lining (29) of the shoe (27) or is configured as part of the inner lining (29) of the shoe (27).

4. Shoe according to one of Claims 1 to 3, wherein the band-like device (32) is configured as a cushion or as a flat band made of a fabric or textile.

5. Shoe according to one of Claims 1 to 4, wherein the shoe (27) has, on the outer side of the shaft, at least one opening, through which one end of the band-like device (32) can be passed from the inside of the shoe (27) to the outside.

6. Shoe according to one of Claims 1 to 5, wherein at least one channel or one or more eyelets or loops are formed in the inner lining (29) or on the inner side of the shoe (27) under the inner lining (29), the band-like device (32) being able to be received therein.

7. Shoe according to one of Claims 1 to 6, wherein the band-like device (32) has two ends, at least one of which branches into two ends, wherein the band-like device (32) has a fastener, with which the two ends of the band-like device (32) can be joined together.

8. Shoe according to one of Claims 1 to 7, wherein at least one end of the band-like device (32) is joined to the lacing.

9. Shoe insole which has a band-like device (32) which is joined to the sole and extends at an angle to the longitudinal direction of the sole, wherein the band-like device (32) is designed, when it is arranged in a shoe (27), to extend in a direction from the upper shaft end to the shoe base and runs in the direction from the from the upper shaft end to the shoe base along at least one muscle fascia (1, 3, 5, 7) of a foot (9) arranged in the shoe, extending from the inner side of the longitudinal arch of the foot toward the lower leg, the band-like device (32) being arranged at the shoe insole or integrated into it to cross the shoe base at the arch of the foot at the level of the anterior lower ankle joint, in order to exert pressure on the at least one muscle fascia (1, 3, 5, 7) of the foot (9) arranged in the shoe (27).

10. Shoe insole according to Claim 9, wherein the band-like device (32) or one or more sections thereof extend(s) at an angle of between 20° and 90°, in particular 30° and 80°, 40° and 70°, 50° and 60° or 60° and 70° to the longitudinal direction of the sole.

11. Sock comprising a band-like device (39) which extends on an inner side of the sock (37) in a direction from the upper end to the foot part of the sock (37) in such a manner that it extends in the direction from the from the upper end to the foot part of the sock (37) along at least one muscle fascia (1, 3, 5, 7) of a foot (9) arranged in the sock, extending from the inner side of the longitudinal arch of the foot toward the lower leg, and crosses the underside of the foot at the arch at the level of the anterior lower ankle joint in order to exert pressure on the at least one muscle fascia (1, 3, 5, 7) of the foot (9) arranged in the sock (37).

12. Sock according to Claim 11, wherein the band-like device (39) is arranged in the sock (37) such that it crosses the foot part of the sock (37) at the arch of the foot.

13. Sock according to one of Claims 11 or 12, wherein the band-like device (39) is arranged in the sock (37) such that it crosses the foot part starting from the location of the base (13) of the fifth metatarsal of the foot (9) in the sock (37).

## Revendications

1. Chaussure qui comprend un dispositif en forme de bande (32) qui est fixé à un côté interne de la chaussure (27) de façon à s'étendre dans la direction de l'extrémité supérieure de la tige vers le fond de la chaussure le long d'au moins un fascia musculaire (1, 3, 5, 7) d'un pied (9) disposé dans la chaussure, s'étendant du côté interne de la voûte plantaire du pied en direction de la jambe, afin d'exercer une pression, dans laquelle le dispositif en forme de bande traverse le fond de la chaussure conformément au fascia musculaire (1, 3, 5, 7) au niveau de la voûte plantaire à la hauteur de l'articulation de cheville inférieure avant.

2. Chaussure selon la revendication 1, dans laquelle le dispositif en forme de bande (32) est disposé dans la chaussure (27) de façon à traverser le fond de la chaussure à partir de la position de la base (13) du cinquième métatarsien du pied (9) dans la chaussure (27).

3. Chaussure selon l'une des revendications 1 ou 2, dans laquelle le dispositif en forme de bande (32) est disposé au moins partiellement sous la garniture interne (29) de la chaussure (27) ou est conçu comme une partie de la garniture interne (29) de la chaussure (27).

4. Chaussure selon l'une des revendications 1 à 3, dans laquelle le dispositif en forme de bande (32) est conçu comme un rembourrage ou comme une bande plate en tissu ou textile.

5. Chaussure selon l'une des revendications 1 à 4, dans laquelle la chaussure (27) comprend, sur le côté externe de la tige, au moins une ouverture à travers laquelle une extrémité du dispositif en forme de bande (32) peut être guidée du côté interne de la chaussure (27) vers le côté externe.

6. Chaussure selon l'une des revendications 1 à 5, dans laquelle, dans la garniture interne (29) ou sur le côté interne de la chaussure (27), sous la garniture interne (29), sont formés au moins un canal ou un ou plusieurs œillets ou boucles, dans lequel resp. dans lesquels le dispositif en forme de bande (32) peut être logé.

7. Chaussure selon l'une des revendications 1 à 6, dans laquelle le dispositif en forme de bande (32) comprend deux extrémités parmi lesquelles au moins une bifurque en deux extrémités, dans laquelle le dispositif en forme de bande (32) comprend une fermeture avec laquelle les deux extrémités du dispositif en forme de bande (32) peuvent être reliées entre elles.

8. Chaussure selon l'une des revendications 1 à 7, dans laquelle au moins une extrémité du dispositif en forme de bande (32) est reliée avec le laçage.

9. Semelle interne de chaussure qui comprend un dispositif en forme de bande (32) qui est reliée avec la semelle et qui s'étend avec un angle par rapport à la direction longitudinale de la semelle, dans laquelle le dispositif en forme de bande (32) est conçu de façon à ce que, lorsqu'il est disposé dans une chaussure (27), il s'étende dans une direction de l'extrémité supérieure de la tige vers le fond de la chaussure et donc s'étend dans la direction de l'extrémité supérieure de la tige vers le fond de la chaussure le long d'au moins un fascia musculaire (1, 3, 5, 7) d'un pied (9) disposé dans la chaussure, s'étendant du côté interne de la voûte plantaire du pied en direction de la jambe, dans laquelle le dispositif en forme de bande (32) est disposé sur la semelle interne de la chaussure ou intégré dans celle-ci de façon à ce qu'il traverse le fond de la chaussure au niveau de la voûte plantaire à la hauteur de l'articulation de cheville inférieure avant, afin d'exercer une pression sur l'au moins un fascia musculaire (1, 3, 5, 7) du pied (9) disposé dans la chaussure (27).

10. Semelle interne de chaussure selon la revendication 9, dans laquelle le dispositif en forme de bande (32) ou une ou plusieurs portions de celui-ci s'étendent avec un angle entre 20° et 90°, plus particulièrement entre 30° et 80°, 40° et 70°, 50° et 60° ou 60° et 70° par rapport à la direction longitudinale de la semelle interne.

11. Chaussette qui comprend un dispositif en forme de bande (39) qui s'étend sur un côté interne de la chaussette (37) dans une direction de l'extrémité supérieur vers la partie de pied de la chaussette (37), de façon à s'étendre dans la direction de l'extrémité supérieure vers la partie de pied de la chaussette le long d'au moins un fascia musculaire (1, 3, 5, 7) d'un pied (9) disposé dans la chaussette, s'étendant du côté interne de la voûte plantaire du pied en direction de la jambe et de façon à traverser le côté inférieur du pied au niveau de la voûte plantaire à la hauteur de l'articulation de cheville inférieure avant, afin d'exercer une pression sur l'au moins un fascia musculaire (1, 3, 5, 7) du pied (9) disposé dans la chaussette (37).

12. Chaussette selon la revendication 11, dans laquelle le dispositif en forme de bande (39) est disposé dans la chaussette (37) de façon à traverser la partie de pied de la chaussette (37) au niveau de la voûte plantaire.

13. Chaussette selon l'une des revendications 11 ou 12, dans laquelle le dispositif en forme de bande (39) est disposé dans la chaussette (37) de façon à traverser la partie de pied à partir de la base (13) du cinquième métatarsien du pied (9) dans la chaussette (37).
